# EUROPÄISCHE PATENTSCHRIFT

(11) **EP 1 263 707 B1**
(45) Veröffentlichungstag und Bekanntmachung des Hinweises auf die Patenterteilung: **09.11.2005**
(21) Anmeldenummer: 01925346.7
(22) Anmeldetag: 21.02.2001
(51) Int. Cl.: C07C 51/235, C07C 53/124, C07C 53/126

(54) **VERFAHREN ZUR HERSTELLUNG ALIPHATISCHER CARBONSÄUREN AUS ALDEHYDEN**
METHOD FOR THE SYNTHESIS OF ALIPHATIC CARBOXYLIC ACIDS FROM ALDEHYDES
PROCEDE DE FABRICATION D'ACIDES CARBOXYLIQUES ALIPHATIQUES A PARTIR D'ALDEHYDES

(30) Priorität: 04.03.2000 DE 10010771
(43) Veröffentlichungstag der Anmeldung: 11.12.2002
(73) Patentinhaber: Celanese Chemicals Europe GmbH, 60439 Frankfurt am Main (DE)
(72) Erfinder: SPRINGER, Helmut, 46539 Dinslaken (DE); LAPPE, Peter, 46539 Dinslaken (DE)
(86) Internationale Anmeldenummer: PCT/EP2001/001943
(87) Internationale Veröffentlichungsnummer: WO 2001/066504

(56) Entgegenhaltungen:
- GB-A- 706 009
- GB-A- 1 103 885
- GB-A- 1 565 716
- US-A- 2 010 358
- US-A- 4 013 691
- US-A- 4 285 875
- US-A- 4 487 720

## Beschreibung

Die vorliegende Erfindung betrifft ein neues, katalytisches Verfahren zur Herstellung aliphatischer Carbonsäuren aus Aldehyden durch Oxidation mit Sauerstoff oder Sauerstoff enthaltenden Gasen.

Aldehyde werden in großem Umfang als Ausgangsstoffe zur Gewinnung von Carbonsäuren eingesetzt. Die Vorzugsstellung für diese Verwendung verdanken Aldehyde ihrer hohen Verfügbarkeit nach einer Reihe, auch industriell genutzter Prozesse. Überdies läßt sich die Carbonylgruppe der Aldehyde leicht in die für Carbonsäuren charakteristische Carboxylgruppe umwandeln. Im Rahmen technisch ausgeübter Verfahren erfolgt die Umsetzung von Aldehyden zu Carbonsäuren häufig in Anwesenheit von Katalysatoren. Es fehlt jedoch nicht an Hinweisen die davor warnen, Katalysatoren einzusetzen, weil sie das Auftreten von Nebenreaktionen, z.B. die Decarbonylierung der eingesetzten Aldehyde zu Kohlenwasserstoffen, fördern. Dementsprechend sind auch unterschiedliche Prozesse bekannt, bei denen auf die Verwendung von Katalysatoren verzichtet wird. Zur Vermeidung von Nebenreaktionen arbeitet man sowohl bei den katalytischen als auch bei den nichtkatalytischen Prozessen bei möglichst niedrigen Temperaturen, im allgemeinen wird eine Reaktionstemperatur von 100°C nicht überschritten. Als Katalysatoren kommen vorwiegend Salze von Übergangsmetallen in Betracht, insbesondere Salze des Kobalts und des Mangans sowie des Chroms, Eisens, Kupfers, Nickels, Silbers und Vanadiums. Dennoch ist die Carbonsäurebildung aus Aldehyden, selbst bei Einhaltung optimaler Temperaturbedingungen, häufig mit Neben- und Abbaureaktionen verbunden. Das gilt gleichermaßen für Reaktionen in Gegenwart wie in Abwesenheit von Katalysatoren. In solchen Fällen kann die Selektivität der Umsetzung durch Zusatz von Alkalimetallsalzen schwacher Säuren zu den Reaktanten erheblich verbessert werden. Nachteilig bei dieser Verfahrensvariante ist jedoch, dass die Alkalimetallsalze inhibierend wirken, so dass für eine vollständige Umsetzung der Ausgangsstoffe lange Reaktionszeiten erforderlich sind.

Nach dem in der DE-OS 30 29 700 beschriebenen Verfahren werden zur Herstellung von aliphatischen Monocarbonsäuren mit 6 bis 9 Kohlenstoffatomen die entsprechenden Aldehyde mit Sauerstoff in reiner Form oder mit Luft oxidiert. Als Katalysator wirkt eine Kombination von Mangan- und Kupferverbindungen, die in der Säure löslich sind. Die Metalle liegen in einer Menge von je etwa 10 bis etwa 2000 ppm, vorzugsweise 200 bis 600 ppm Mangan und Kupfer, bezogen auf das Gewicht des flüssigen Reaktionsgemischs, vor. Das Molverhältnis von Mangan zu Kupfer beträgt 5:1 bis 0,5:1. Die Umsetzung der Ausgangsstoffe erfolgt in flüssiger Phase bei Temperaturen von etwa 50 bis 80°C und Drücken im Bereich von etwa 1,4 bis 10,3 bar. Als Hauptschwierigkeit dieses Verfahrens wird in der Prozessbeschreibung die Anwesenheit von Kupfer- und auch Manganverbindungen im Reaktionsprodukt, d.h. in der Carbonsäure, geschildert. Zur Entfernung der Metalle sind aufwendige Reinigungsmaßnahmen erforderlich, beispielsweise ihre Ausfällung mit wäßriger Oxalsäure.

Das in der US-Patentschrift 4 487 720 offenbarte Verfahren zur Herstellung von C₅- bis C₉-Monocarbonsäuren durch Oxidation von Aldehyden der gleichen Kohlenstoffatom-Zahl mit reinem Sauerstoff oder mit Luft arbeitet ebenfalls mit Kupfer- und Manganverbindungen als Katalysatoren. Die Gesamtmenge der Metalle erstreckt sich über einen Bereich von 10 bis 200 ppm, bezogen auf das Gesamtgewicht der aus Aldehyd, Säure und Katalysator bestehenden Lösung. Mangan und Kupfer werden in einem Molverhältnis von etwa 3:1 bis etwa 1:1 eingesetzt. Als Nachteil dieser Arbeitsweise wird die Bildung von Kupferfilmen beschrieben, die bei der destillativen Reinigung der Säure auftreten und mechanische Schäden in der Destillationsapparatur zur Folge haben. Um dieses Problem zu vermeiden wird empfohlen, die Destillation in Gegenwart von Sauerstoff durchzuführen.

Ein weiteres katalytisches Verfahren zur Reaktion von Aldehyden mit Sauerstoff unter Bildung von Carbonsäuren ist Gegenstand der offengelegten internationalen Anmeldung WO97/14668. Als Katalysatoren finden substituierte oder unsubstituierte Alkylamine, Alkylamin-N-oxide, aromatische Amine, aromatische N-oxide, heterocyclische Amine, heterocyclische Amin-N-oxide und deren Mischungen in einer Menge Verwendung, die von etwa 0,001 oder weniger bis etwa 10 oder mehr Moläquivalente, bezogen auf den Aldehyd, reicht. Bevorzugt werden etwa 0,005 bis etwa 2 Moläquivalente und insbesondere etwa 0,005 bis etwa 1,2 Moläquivalente des Amins bzw. des Amin-N-oxids, bezogen auf den Aldehyd, eingesetzt. Ausdrücklich wird darauf hingewiesen, dass die katalytisch wirkenden Stickstoffverbindungen einen im Vergleich zum Reaktionsprodukt höheren Siedepunkt besitzen müssen, um eine Verunreinigung der Säure durch den Katalysator zu unterbinden.

Nach der Lehre der offengelegten japanischen Patentanmeldung 53-105413 oxidiert man α-verzweigte aliphatische Aldehyde mit Sauerstoff in Gegenwart von Lithium- oder Erdalkalimetallverbindungen, die in Mengen von 0,01 bis 10 Gew.-% (bezogen auf das gesamte Reaktionssystem) eingesetzt werden, um α-verzweigte aliphatische Carbonsäuren herzustellen.

Kennzeichnend für die in der französischen Patentanmeldung 2 769 624 beschriebene Arbeitsweise ist die Einhaltung niedriger Reaktionstemperaturen, nämlich Temperaturen zwischen 0 und 25°C. Das Verfahren erfordert ebenfalls die Gegenwart von Alkalimetall- bzw. Erdalkalimetallverbindungen als Hilfsstoffe. Es bleibt offen, welche speziellen Wirkungen diese Verbindungen entfalten, d.h. ob sie, wie bekannt, lediglich die Selektivität der Umsetzung verbessern oder aber bei den gewählten, niedrigen Temperaturen, möglicherweise auch die Reaktionsgeschwindigkeit erhöhen.

Gegenstand der bekanntgemachten deutschen Patentanmeldung 26 04 545 ist die Herstellung von Alkylcarbonsäuren der allgemeinen Formel CₙH₂ₙ₊₁COOH, in der n für einen Wert von 2 bis 18 steht, durch Hydroformylierung eines Olefins CₙH₂ₙ und unmittelbare Oxidation des bei der Hydroformylierung anfallenden Reaktionsgemisches. Unmittelbar heißt in diesem Zusammenhang, dass eine vorherige Aufarbeitung des Hydroformylierungsproduktes unterbleibt. Das Verfahren dient insbesondere zur Herstellung von Gemischen isomerer C₉- bis C₁₆-Fettsäuren. Als Ausgangsolefine kommen bevorzugt Dimere und Trimere des Propens und der Butene in Betracht, darunter vor allem das dimere Isobuten (2,4,4-Trimethylpenten-1). Beide Einzelumsetzungen des zweistufigen Verfahrens, d.h. sowohl die Hydroformylierung als auch die Oxidation werden durch Rhodium in Form seiner Verbindungen katalysiert. Maßgebend für die Rhodium-Konzentration in dem der Oxidation unterworfenen Reaktionsgemisch ist daher der relativ hohe Rhodiumanteil im Hydroformylierungsprodukt. Um die Wirtschaftlichkeit des Gesamtprozesses sicherzustellen ist es erforderlich, das Edelmetall durch geeignete Maßnahmen möglichst vollständig aus dem Endprodukt des Prozesses, der Carbonsäure, wiederzugewinnen. Überdies ist nicht auszuschließen, dass durch Rhodium in der vorliegenden Konzentration während des Oxidationsvorganges unerwünschte Nebenreaktionen begünstigt werden, denn die Carbonsäure-Ausbeute ist, wie die Beispiele zeigen, für eine technische Nutzung des Verfahrens unzureichend.

LARKIN berichtet in J.Org.Chem. 1990, 55, S. 1563 ff., dass die Gegenwart von Katalysatoren bei der kommerziell ausgeführten Oxidation von Aldehyden zu Carbonsäuren deshalb für notwendig erachtet wird, weil im Reaktionsgemisch Spuren von Metallsalzen enthalten sind, die Nebenreaktionen katalysieren können. Die Bildung der Metallsalze geht auf die Korrosion metallischer Anlagenteile zurück. Aufgabe der Katalysatoren ist es, die Wirkung der Korrosionsprodukte zu überkompensieren.

Auch in Ullmanns Encyklopädie der technischen Chemie, 4. Auflage 1972 ff., Band 9, wird mehrfach auf den negativen Einfluss metallischer Verunreinigungen in den zur Oxidation eingesetzten Ausgangsaldehyden hingewiesen. So führen z.B. im Butyraldehyd gelöste Eisen- und Kobaltsalze bei dessen Oxidation zu Buttersäure zu einem erhöhten Anfall von Nebenprodukten (l.c., Seite 142, linke Spalte) und bei der Oxidation von 2-Ethylhexanal zu 2-Ethylhexansäure beschleunigen Schwermetallionen die Decarbonylierung des Ausgangsaldehyds zu Heptan (l.c. Seite 144, linke Spalte).

Die bekannten Verfahren zur Herstellung von Carbonsäuren aus Aldehyden erfüllen noch nicht im vollen Umfang die technischen und wirtschaftlichen Erfordernisse, die an moderne, industriell genutzte Prozesse gestellt werden. Die Anwendung von Katalysatoren hat oftmals das Auftreten unerwünschter Nebenreaktionen zur Folge. Darüber hinaus erfordern sie auch aufwendige Reinigungsschritte, denen das Reaktionsprodukt unterworfen werden muss, um Carbonsäuren zu erhalten, die problemlos weiterverarbeitet werden können. Nichtkatalytische Prozesse befriedigen häufig nicht hinsichtlich Reaktionsgeschwindigkeit und bezüglich Umsatz und Selektivität zum gewünschten Produkt.

Es bestand daher die Aufgabe eine Arbeitsweise zu entwickeln, die die Vorteile der nichtkatalytischen Oxidation von Aldehyden zu Carbonsäure, insbesondere die Abwesenheit von störenden Fremdstoffen und die Vermeidung von Nebenreaktionen, mit den Vorteilen der Oxidation in Gegenwart von Katalysatoren, vor allem eine ausreichende Reaktionsgeschwindigkeit, verbindet, die Nachteile der jeweiligen Umsetzungen jedoch weitgehend ausschließt. Als Ergebnis wird die Gewinnung von Carbonsäuren aus Aldehyden mit vertretbarem technischen Aufwand in hoher Ausbeute und Reinheit angestrebt.

Diese Aufgabe wird gelöst durch ein Verfahren zur Herstellung aliphatischer Carbonsäuren mit 4 bis 11 Kohlenstoffatomen durch Oxidation der entsprechenden Aldehyde mit Sauerstoff oder Sauerstoff enthaltenden Gasgemischen bei 20 bis 100°C. Es ist dadurch gekennzeichnet, dass die Oxidation der Aldehyde in Gegenwart von 0,1 bis 5,0 Gew.-ppm eines Metalls der Gruppen 5 bis 11 des Periodensystems der Elemente oder der entsprechenden Menge einer Verbindung eines solchen Metalls oder Gemischen solcher Metalle und/oder Metallverbindungen, bezogen auf eingesetzten Aldehyd, erfolgt, und wobei der Zusatz mehrzähniger Liganden, die stickstoffhaltige Heterocyclen enthalten, ausgeschlossen wird.

Überraschenderweise gelingt es, in Gegenwart geringer Mengen ausgewählter Metalle oder Verbindungen dieser Metalle, Aldehyde mit reinem Sauerstoff oder Sauerstoff enthaltenden Gasgemischen mit hohem Umsatz und sehr selektiv zu den entsprechenden Carbonsäuren umzusetzen. Die angewandten Metallmengen, sie betragen maximal 5 Gew.-Teile je 1 Million Gew.-Teile Aldehyd stellen eine, auch für technische Bedürfnisse, ausreichende Reaktionsgeschwindigkeit sicher. Sie geben jedoch nicht zu unerwünschten Nebenreaktionen Anlass, so dass die Aldehyde nahezu ausschließlich in die ihnen entsprechenden Carbonsäuren umgewandelt werden. Überdies sind die eingesetzten Metallmengen so gering, dass sie weder unter dem Aspekt der Wirtschaftlichkeit des Verfahrens, z.B. bei Verwendung teurer Edelmetalle, noch im Hinblick auf die für die verschiedenen Anwendungsgebiete geforderte Reinheit der Carbonsäuren, aus dem Reaktionsprodukt wiedergewonnen bzw. entfernt werden müssen.

Als Katalysator wird dem Oxidationsgemisch erfindungsgemäß mindestens ein Metall aus den Gruppen 5 bis 11 des Periodensystems der Elemente (in der Fassung der IUPAC-Empfehlung von 1985) oder mindestens eine Verbindung eines solchen Metalls, zugesetzt. Verwendet man Metalle als Katalysatoren, so empfiehlt es sich, sie in feiner Verteilung dem Reaktionsgemisch hinzuzufügen, um ihre Umwandlung in die katalytisch aktive Form zu erleichtern. Denn es kann angenommen werden, dass das Metall mit im Aldehyd in Spuren vorhandener Carbonsäure unter Bildung eines im Reaktionsgemisch löslichen, katalytisch wirkenden Salzes reagiert. Statt der Metalle in elementarer Form können auch Verbindungen der Metalle als Katalysatoren Anwendung finden. Die Art der Verbindungen unterliegt dabei keiner Beschränkung. Sofern nicht besondere Gründe vorliegen, wird man jedoch solche Verbindungen bevorzugen, die im Reaktionsmedium von Anfang an löslich sind, um eine Verzögerung des Reaktionseintritts durch vorherige Bildung einer löslichen und damit besonders aktiven Metallverbindung zu vermeiden.

Zu den katalytisch, bereits in sehr geringer Menge wirksamen Metallen der 5. bis 11. Gruppe zählen Vanadium, Chrom, Molybdän, Eisen, Kobalt, Nickel, Ruthenium, Rhodium, Palladium, Kupfer, vorzugsweise Chrom, Eisen, Nickel, Rhodium und insbesondere Eisen und Rhodium. Als im Reaktionsgemisch lösliche Verbindungen verwendet man Salze, insbesondere Salze organischer Säuren, wobei Carboxylate der Säuren bevorzugt werden, die das Ergebnis der Oxidationsreaktion sind. Andere geeignete Verbindungen der erfindungsgemäß eingesetzten Metalle sind Komplexverbindungen, z.B. Acetylacetonate, Metallcarbonyle, Hydridometallcarbonyle, ferner Carbonylverbindungen, die neben Kohlenmonoxid und gegebenenfalls Wasserstoff noch weitere Liganden, z.B. durch organische Reste substituierte Phosphine wie Arylphosphine, Alkylphosphine, Arylalkylphosphine enthalten. Ein Beispiel für derartige Liganden ist das Triphenylphosphin.

Es ist nicht erforderlich, die katalytisch wirksamen Metalle oder die katalytisch wirksamen Metalle enthaltenden Verbindungen als reine Substanzen einzusetzen. Vielmehr können auch Gemische der genannten Metalle bzw. der Metallverbindungen und ebenso auch Gemische aus Metallen und Metallverbindungen als Katalysatoren eingesetzt werden.

Neben der Auswahl der katalytisch wirksamen Metalle ist ein weiteres sehr wesentliches Merkmal des neuen Verfahrens die Einhaltung eines maximalen Gewichtsverhältnisses zwischen Katalysator und zu oxidierendem Aldehyd. Erfindungsgemäß ist die obere Grenze dieses Verhältnisses 5 ppm, d.h. 5 Gew.-Teil Katalysatormetall je 10⁶ Gew.-Teile Aldehyd. Es hat sich besonders bewährt, auf 10⁶ Gew.-Teile Aldehyd 0,2 bis 3 Gew.-Teile Katalysatormetall und vorzugsweise 0,5 bis 2 Gew.-Teile Katalysatormetall anzuwenden. Die vorstehend beschriebenen Verhältnisse zwischen Katalysatormetall und Aldehyd gelten auch bei Verwendung von Metallverbindungen, d.h. die Menge der einzusetzenden Verbindung bemisst sich nach ihrem Metallgehalt. Entsprechendes gilt bei Einsatz von Mischungen verschiedener katalytisch wirksamer Metalle bzw. Metallverbindungen und von Mischungen aus Metallen und Metallverbindungen.

Das Verfahren der Erfindung wird im Temperaturbereich von 20 bis 100°C durchgeführt. Vorzugsweise arbeitet man zwischen 20 und 80°C, insbesondere zwischen 40 und 80°C. Die Temperaturführung, konstante oder variable Temperatur, kann den individuellen Erfordernissen des Ausgangsmaterials und den Reaktionsgegebenheiten angepasst werden.

Die Umsetzung der Reaktionspartner erfolgt bevorzugt bei Atmosphärendruck. Die Anwendung erhöhten Drucks ist jedoch nicht ausgeschlossen. Üblicherweise arbeitet man in einem Bereich von Atmosphärendruck bis 1,0 MPa, vorzugsweise bei Atmosphärendruck bis 0,8 MPa.

Die zur Umwandlung von Aldehyden in Carbonsäuren nach dem erfindungsgemäßen Verfahren erforderliche Reaktionszeit hängt unter anderem ab von der Reaktionstemperatur, der Art der Einsatzstoffe und dem Mengenverhältnis der Reaktanten zueinander. Normalerweise beträgt sie 30 min bis 20 h, insbesondere 3 bis 8 h.

Im Mittelpunkt des neuen Prozesses steht die Oxidation von C₄- bis C₁₁-Aldehyden, sowohl unverzweigten als auch verzweigten. Die Herkunft der Aldehyde ist nicht auf bestimmte Herstellungsverfahren beschränkt. Aufgrund ihrer leichten Zugänglichkeit werden durch Oxosynthese, d.h. durch Reaktion von C₃- bis C₁₀-Olefinen mit Kohlenmonoxid und Wasserstoff gewonnene Aldehyde bevorzugt. In diesem Zusammenhang ist es nicht entscheidend, welche spezielle Ausführungsform der Oxosynthese zur Gewinnung der Aldehyde diente, d.h. ob die Reaktion z.B. durch Kobalt oder durch Rhodium katalysiert wurde, ob man die Metalle allein oder zusammen mit Komplexbildnern einsetzte und der Katalysator im Reaktionsgemisch homogen gelöst war oder eine eigene, heterogene Phase bildete.

Als Oxidationsmittel verwendet man nach dem Verfahren der Erfindung molekularen Sauerstoff oder Gasgemische, die molekularen Sauerstoff enthalten. Weitere Bestandteile derartiger Gasgemische sind inerte Gase, z.B. Stickstoff, Edelgase und Kohlendioxid. Der Anteil der inerten Bestandteile des Sauerstoff enthaltenden Gasgemisches beträgt bis zu 90 Vol-%, insbesondere 30 bis 80 Vol-%. Die bevorzugten Oxidationsmittel sind Sauerstoff oder Luft.

Die Aldehyde können als solche oder gelöst in einem, unter den Reaktionsbedingungen inerten, Lösungsmittel eingesetzt werden. Beispiele für geeignete Lösungsmittel sind Ketone wie Aceton, Ester, z.B. Ethylacetat, Kohlenwasserstoffe, z.B. Toluol und Nitrokohlenwasserstoffe wie Nitrobenzol. Die Konzentration des Aldehyds wird durch seine Löslichkeit in dem Lösungsmittel begrenzt.

Das erfindungsgemäße Verfahren kann absatzweise oder kontinuierlich durchgeführt werden. Eine Rückführung nicht umgesetzter Reaktionsteilnehmer ist in beiden Fällen möglich.

Nach einer bewährten Ausführungsform des erfindungsgemässen Verfahrens legt man den Aldehyd zusammen mit dem Katalysator in einem geeigneten Reaktor, z.B. einem mit einem Anströmboden versehenen Rohrreaktor, der gegebenenfalls noch Füllkörper enthält, vor und leitet den Sauerstoff oder das Sauerstoff enthaltende Gasgemisch von unten durch den, Katalysator gelöst oder suspendiert enthaltenden Aldehyd.

Entsprechend einer weiteren Ausführungsform verwendet man als Reaktor einen Rieselturm, der Füllkörper enthält. Über die Füllung läßt man Aldehyd und Katalysator herabrieseln und leitet in den Turm gleichzeitig im Gleich- oder Gegenstrom, Sauerstoff oder ein Sauerstoff enthaltendes Gasgemisch ein.

In den folgenden Beispielen wird die Herstellung von n-Buttersäure, 2-Methylbuttersäure, n-Heptansäure und Isononansäure nach dem beanspruchten Verfahren beschrieben. Die Umsetzung der Ausgangsaldehyde erfolgt entsprechend der Erfindung in Gegenwart von Metallen der 5. bis 11. Gruppe des Periodensystems oder Verbindungen dieser Metalle als Katalysatoren. Den Beispielen werden die Ergebnisse von Versuchen (Vergleichsbeispiele) gegenübergestellt, in denen die Aldehyde nichtkatalytisch oxidiert wurden. Eine Ausnahme stellt das Vergleichsbeipiel 3 dar, das die Oxidation von 2-Methylbutanal beschreibt. Unter Berücksichtigung der Eigenart α-verzweigter Aldehyde bei der nichtkatalytischen Oxidation verstärkt Nebenreaktionen einzugehen, wurde in diesem Beispiel die vom Fachmann üblicherweise gewählte Oxidation in Gegenwart eines Alkalisalzes als Vergleich herangezogen. Die jeweiligen Versuchsergebnisse werden durch Angabe folgender Kenngrößen wiedergegeben:
- GC-Analyse der Rohsäure; die Vorlaufkomponente ist nicht aufgegliedert, sondern unter der Bezeichnung Leichtsieder zusammengefasst;
- Aldehyd-Umsatz;
- Selektivität; sie ergibt sich aus dem Carbonsäure-Anteil im Reaktionsprodukt, bezogen auf umgesetzten Aldehyd.

Selbstverständlich ist das neue Verfahren nicht auf die nachstehend beschriebenen Ausführungsformen beschränkt.

### Beispiele

### Herstellung von n-Buttersäure

### Vergleichsbeispiel 1

Die Flüssigphasenoxidation von n-Butanal zu n-Buttersäure wurde ohne Katalysatorzusatz in einem Blasensäulen-Reaktor aus Glas mit einem Innendurchmesser von 38 mm und 150 cm Länge durchgeführt. Abhängig vom Reaktionsverhalten wurde der Reaktor mantelseitig durch einen mit einem Wärmeaustauscher verbundenen Wasserkreislauf gekühlt oder beheizt und die Innentemperatur auf diese Weise konstant gehalten. Die Sauerstoffzufuhr erfolgte von unten durch eine mit der Blasensäule verbundene Glasfilterplatte mit einer maximalen Porenweite von 16-40µm.

In die Oxidation wurden 800,0 g Aldehyd eingesetzt. Nach 6 Stunden Oxidation bei konstant 40°C wurden folgende Ergebnisse ermittelt:

| GC-Analyse (%) | |
|---|---|
| Leichtsieder | 0,10 |
| n-Butanal | 2,76 |
| n-Buttersäure | 96,80 |
| Sonstige | 0,34 |
| | |
| Umsatz n-Butanal (% d.Th.) | 96,6 |
| Selektivität zu n-Buttersäure (% d.Th.) | 99,6 |

### Beispiel 1

In einem 150 ml-Stahlautoklaven wurde eine Lösung aus 44,6 g Toluol, 5,09 g Triphenylphosphin und 20 mg Rhodium (in Form von Rh-2-ethylhexanoat) 60 min bei 110°C unter einem Druck von 27 MPa mit Synthesegas behandelt. 2,7 g der resultierenden Lösung mit einem Gehalt von 1,1 mg Rhodium wurden mit 800,0 g Butanal gemischt und unter den Bedingungen des Vergleichsbeispiels 1 in die Oxidation eingesetzt.

Nach 6 Stunden Oxidation bei konstant 40°C wurden folgende Ergebnisse ermittelt:

| GC-Analyse (%) | |
|---|---|
| Leichtsieder | 0,17 |
| Toluol | 0,25 |
| n-Butanal | 0,49 |
| n-Buttersäure | 98,82 |
| Sonstige | 0,27 |
| | |
| Umsatz n-Butanal (% d.Th.) | 99,4 |
| Selektivität zu n-Buttersäure (% d.Th.) | 99,4 |

### Herstellung von 2-Methylbuttersäure

### Vergleichsbeispiel 2

Die Flüssigphasenoxidation von 2-Methylbutanal zu 2-Methylbuttersäure wurde ohne Katalysatorzusatz in einem Blasensäulen-Reaktor aus Glas mit einem Innendurchmesser von 38 mm und 150 cm Länge durchgeführt. Abhängig vom Reaktionsverhalten wurde der Reaktor mantelseitig durch einen mit einem Wärmeaustauscher verbundenen Wasserkreislauf gekühlt oder beheizt und die Innentemperatur auf diese Weise konstant gehalten. Die Sauerstoffzufuhr erfolgte von unten durch eine mit der Blasensäule verbundene Glasfilterplatte mit einer maximalen Porenweite von 16-40µm.

In die Oxidation wurden 800,0 g Aldehyd eingesetzt. Nach 6 Stunden Oxidation bei konstant 50°C wurden folgende Ergebnisse ermittelt:

| GC-Analyse (%) | |
|---|---|
| Leichtsieder | 0,79 |
| 2-Methylbutanal | 1,84 |
| 2-Methylbuttersäure | 85,53 |
| Sonstige | 11,84 |
| | |
| Umsatz 2-Methylbutanal (% d.Th.) | 97,5 |
| Selektivität zu 2-Methylbuttersäure (% d.Th.) | 85,9 |

### Vergleichsbeispiel 3

Unter den Bedingungen von Vergleichsbeispiel 2 wurden 800 g 2-Methylbutanal zusammen mit einem Gemisch aus 75,3 g 2-Methylbuttersäure sowie 20,7 g 50 Gew.-%iger wässriger Kalilauge (entsprechend 2 mol-% Kalium, bezogen auf 2-Methylbutanal) in die Oxidation eingesetzt.

Nach 6 Stunden Oxidation bei konstant 50°C wurden folgende Ergebnisse ermittelt:

| GC-Analyse (%) | |
|---|---|
| Leichtsieder | 2,01 |
| 2-Methylbutanal | 2,45 |
| 2-Methylbuttersäure | 93,63 |
| Sonstige | 1,91 |
| | |
| Umsatz 2-Methylbutanal (% d.Th.) | 96,5 |
| Selektivität zu 2-Methylbuttersäure (% d.Th.) | 95,0 |

### Beispiel 2

In einem 150 ml-Stahlautoklaven wurde eine Lösung aus 44,0 g Toluol und 22 mg Rhodium (in Form von Rh-2-ethylhexanoat) 60 min bei 120°C unter einem Druck von 27 MPa mit Synthesegas behandelt. 3,81 g der resultierenden Lösung mit einem Gehalt von 1,9 mg Rhodium wurden mit 800,0 g 2-Methylbutanal gemischt und unter den Bedingungen des Vergleichsbeispiels 3, d.h. in Gegenwart von 75,3 g 2-Methylbuttersäure sowie 20,7 g 50-Gew.%iger wässriger Kalilauge, in die Oxidation eingesetzt.

Nach 6 Stunden Oxidation bei konstant 50°C wurden folgende Ergebnisse ermittelt:

| GC-Analyse (%) | |
|---|---|
| Leichtsieder | 1,39 |
| Toluol | 0,43 |
| 2-Methylbutanal | 1,28 |
| 2-Methylbuttersäure | 94,38 |
| Sonstige | 2,52 |
| | |
| Umsatz 2-Methylbutanal (% d.Th.) | 98,1 |
| Selektivität zu 2-Methylbuttersäure (% d.Th.) | 95,1 |

### Beispiel 3

Analog Vergleichsbeispiel 2 wurden 800,0 g 2-Methylbutanal zusammen mit einem Gemisch aus 75,3 g 2-Methylbuttersäure und 20,7 g 50 Gew.-%iger Kalilauge in die Oxidation eingesetzt. Die Methylbuttersäure enthielt, als Salze gelöst, 0,10 mg Chrom, 0,07 mg Nickel und 0,47 mg Eisen.

Nach 6 Stunden Oxidation bei konstant 50°C wurden folgende Ergebnisse ermittelt:

| GC-Analyse (%) | |
|---|---|
| Leichtsieder | 1,41 |
| 2-Methyfbutanal | 1,17 |
| 2-Methylbuttersäure | 94,93 |
| Sonstige | 2,49 |
| | |
| Umsatz 2-Methylbutanal (% d.Th.) | 98,3 |
| Selektivität zu 2-Methylbuttersäure (% d.Th.) | 95,3 |

### Herstellung von n-Heptansäure

### Vergleichsbeispiel 4

Die Flüssigphasenoxidation von n-Heptanal zu n-Heptansäure wurde ohne Katalysatorzusatz in einem Blasensäulen-Reaktor aus Glas mit einem Innendurchmesser von 38 mm und 150 cm Länge durchgeführt. Abhängig vom Reaktionsverhalten wurde der Reaktor mantelseitig durch einen mit einem Wärmeaustauscher verbundenen Wasserkreislauf gekühlt oder beheizt und die Innentemperatur auf diese Weise konstant gehalten. Die Sauerstoffzufuhr erfolgte von unten durch eine mit der Blasensäule verbundene Glasfilterplatte mit einer maximalen Porenweite von 16-40µm.

In die Oxidation wurden 800,0 g Aldehyd eingesetzt. Nach 6 Stunden Oxidation bei konstant 50°C wurden folgende Ergebnisse ermittelt:

| GC-Analyse (%) | |
|---|---|
| Leichtsieder | 0,82 |
| n-Heptanal | 5,42 |
| n-Heptansäure | 91,79 |
| Sonstige | 1,97 |
| | |
| Umsatz n-Heptanal (% d.Th.) | 93,8 |
| Selektivität zu n-Heptansäure (% d.Th.) | 98,9 |

### Beispiel 4

In einem 150 ml-Stahlautoklaven wurde eine Lösung aus 44,6 g Toluol, 5,09 g Triphenylphosphin und 20 mg Rhodium (in Form von Rh-2-ethylhexanoat) 60 min bei 110°C unter einem Druck von 27 MPa mit Synthesegas behandelt. 1,78 g der resultierenden Lösung mit einem Gehalt von 0,7 mg Rhodium wurden mit 800,0 g n-Heptanal gemischt und unter den Bedingungen des Vergleichsbeispiels 4 in die Oxidation eingesetzt.

Nach 6 Stunden Oxidation bei konstant 50°C wurden folgende Ergebnisse ermittelt:

| GC-Analyse (%) | |
|---|---|
| Leichtsieder | 0,53 |
| Toluol | 0,26 |
| n-Heptanal | 2,28 |
| n-Heptansäure | 95,19 |
| Sonstige | 1,74 |
| | |
| Umsatz n-Heptanal (% d.Th.) | 97,4 |
| Selektivität zu n-Heptansäure (% d.Th.) | 97,4 |

### Beispiel 5

In einem 150 ml-Stahlautoklaven wurde eine Lösung aus 44,6 g Toluol und 20 mg Rhodium (in Form von Rh-2-ethylhexanoat) 60 min bei 110°C unter einem Druck von 27 MPa mit Synthesegas behandelt. 1,78 g der resultierenden Lösung mit einem Gehalt von 0,8 mg Rhodium wurden mit 800,0 g n-Heptanal gemischt und unter den Bedingungen des Vergleichsbeispiels 4 in die Oxidation eingesetzt.

Nach 6 Stunden Oxidation bei konstant 50°C wurden folgende Ergebnisse ermittelt:

| GC-Analyse (%) | |
|---|---|
| Leichtsieder | 0,25 |
| Toluol | 0,25 |
| n-Heptanal | 1,99 |
| n-Heptansäure | 95,15 |
| Sonstige | 2,36 |
| | |
| Umsatz n-Heptanal (% d.Th.) | 97,7 |
| Selektivität zu n-Heptansäure (% d.Th.) | 97,7 |

### Herstellung von Isononansäure

### Vergleichsbeispiel 5

Die Flüssigphasenoxidation von Isononaldehyd zu Isononansäure wurde ohne Katalysatorzusatz in einem Blasensäulen-Reaktor aus Glas mit einem Innendurchmesser von 38 mm und 150 cm Länge durchgeführt. Abhängig vom Reaktionsverhalten wurde der Reaktor mantelseitig durch einen mit einem Wärmeaustauscher verbundenen Wasserkreislauf gekühlt oder beheizt und die Innentemperatur auf diese Weise konstant gehalten. Die Sauerstoffzufuhr erfolgte von unten durch eine mit der Blasensäule verbundene Glasfilterplatte mit einer maximalen Porenweite von 16-40µm.

In die Oxidation wurden 800,0 g Aldehyd eingesetzt. Nach 6 Stunden Oxidation bei konstant 50°C wurden folgende Ergebnisse ermittelt:

| GC-Analyse (%) | |
|---|---|
| Leichtsieder | 3,78 |
| Isononaldehyd | 8,46 |
| Isononansäure | 84,66 |
| Sonstige | 3,10 |
| | |
| Umsatz lsononaldehyd (% d.Th.) | 90,0 |
| Selektivität zu Isononansäure (% d.Th.) | 99,9 |

### Beispiel 6

800,0 g Isononaldehyd wurden mit einer Lösung aus 1,35 g Toluol und 0,6 mg Rhodium (in Form von Rh-2-ethylhexanoat) gemischt und unter den Bedingungen des Vergleichsbeispiels 5 in die Oxidation eingesetzt.

Nach 6 Stunden Oxidation bei konstant 50°C wurden folgende Ergebnisse ermittelt:

| GC-Analyse (%) | |
|---|---|
| Leichtsieder | 4,19 |
| Toluol | 0,24 |
| Isononaldehyd | 1,65 |
| Isononansäure | 90,05 |
| Sonstige | 3,87 |
| | |
| Umsatz Isononaldehyd (% d.Th.) | 98,0 |
| Selektivität zu Isononansäure (% d.Th.) | 98,4 |

## Patentansprüche

1. Verfahren zur Herstellung aliphatischer Carbonsäuren mit 4 bis 11 Kohlenstoffatomen durch Oxidation der entsprechenden Aldehyde mit Sauerstoff oder Sauerstoff enthaltenden Gasgemischen bei 20 bis 100°C, **dadurch gekennzeichnet, dass** die Oxidation der gereinigten Aldehyde in Gegenwart von 0,1 bis 5 Gew.-ppm eines Metalls der Gruppen 5 bis 11 des Periodensystems der Elemente oder der entsprechenden Menge einer Verbindung eines solchen Metalls oder Gemischen solcher Metalle und/oder Metallverbindungen, bezogen auf eingesetzten Aldehyd, erfolgt, und wobei der Zusatz mehrzähniger Liganden, die stickstoffhaltige Heterocyclen enthalten, ausgeschlossen wird.

2. Verfahren nach Anspruch 1, **dadurch gekennzeichnet, dass** die Oxidation der Aldehyde in Gegenwart von 0,2 bis 3 ppm, insbesondere von 0,5 bis 2 ppm eines Metalls der Gruppen 5 bis 11 des Periodensystems der Elemente oder der entsprechenden Menge einer Verbindung eines solchen Metalls oder Gemischen solcher Metalle und/oder Metallver bindungen, bezogen auf eingesetzten Aldehyd, erfolgt.

3. Verfahren nach Anspruch 1 oder 2, **dadurch gekennzeichnet, dass** das Metall der 5. bis 11. Gruppe des Periodensystems der Elemente Vanadium, Chrom, Molybdän, Eisen, Kobalt, Nickel, Ruthenium, Rhodium, Palladium oder Kupfer, vorzugsweise Chrom, Eisen, Nickel, Rhodium und insbesondere Eisen und Rhodium ist.

4. Verfahren nach Anspruch 1 oder 2, **dadurch gekennzeichnet, dass** die Metallverbindungen sich von den Metallen Vanadium, Chrom, Molybdän, Eisen, Kobalt, Nickel, Ruthenium, Rhodium, Palladium, Kupfer, vorzugsweise Chrom, Eisen, Nickel, Rhodium, und insbesondere Eisen und Rhodium ableiten.

5. Verfahren nach einem oder mehreren der Ansprüche 1 bis 4, **dadurch gekennzeichnet, dass** die Metallverbindungen Carboxylate, Acetylacetonate oder Carbonylverbindungen sind.

6. Verfahren nach Anspruch 5, **dadurch gekennzeichnet, dass** die Metallcarboxylate Salze der Carbonsäuren sind, die als Ergebnis der Oxidation der eingesetzten Aldehyde entstehen.

7. Verfahren nach einem oder mehreren der Ansprüche 1 bis 6, **dadurch gekennzeichnet, dass** die Oxidation bei Temperaturen im Bereich von 20 bis 80°C, vorzugsweise 40 bis 80°C, durchgeführt wird.

8. Verfahren nach einem oder mehreren der Ansprüche 1 bis 7, **dadurch gekennzeichnet, dass** die Oxidation bei Drücken in einem Bereich von Atmosphärendruck bis 1,0 MPa, vorzugsweise Atmosphärendruck bis 0,8 MPa durchgeführt wird.

9. Verfahren nach einem oder mehreren der Ansprüche 1 bis 8, **dadurch gekennzeichnet, dass** die Sauerstoff enthaltenden Gasgemische einen Anteil von bis zu 90 Vol-%, insbesondere 30 bis 80 Vol-%, inerter Bestandteile aufweisen.

## Claims

1. A process for preparing aliphatic carboxylic acids having 4 to 11 carbon atoms by oxidation of the corresponding aldehydes with oxygen or oxygen-containing gas mixtures at 20 to 100°C, wherein the oxidation of the purified aldehydes takes place in the presence of 0.1 to 5.0 ppm by weight of a metal of groups 5 to 11 of the Periodic Table of the Elements or the corresponding amount of a compound of such a metal or mixtures of such metals and/or metal compounds, based on the aldehyde employed, with the proviso that the addition of polydentate ligands containing nitrogenous heterocycles shall be excluded.

2. The process as claimed in claim 1, wherein the oxidation of the aldehydes takes place in the presence of from 0.2 to 3 ppm, in particular from 0.5 to 2 ppm, of a metal of groups 5 to 11 of the Periodic Table of the Elements or the corresponding amount of a compound of such a metal or mixtures of such metals and/or metal compounds, based on the aldehyde employed.

3. The process as claimed in claim 1 or 2, wherein the metal of groups 5 to 11 of the Periodic Table of the Elements is vanadium, chromium, molybdenum, iron, cobalt, nickel, ruthenium, rhodium, palladium or copper, preferably chromium, iron, nickel, rhodium and, in particular, iron and rhodium.

4. The process as claimed in claim 1 or 2, wherein the metal compounds are derived from the metals vanadium, chromium, molybdenum, iron, cobalt, nickel, ruthenium, rhodium, palladium, copper, preferably chromium, iron, nickel, rhodium and, in particular, iron and rhodium.

5. The process as claimed in one or more of claims 1 to 4, wherein the metal compounds are carboxylates, acetylacetonates or carbonyl compounds.

6. The process as claimed in claim 5, wherein the metal carboxylates are salts of the carboxylic acids produced as the result of the oxidation of the aldehydes employed.

7. The process as claimed in one or more of claims 1 to 6, wherein the oxidation is carried out at temperatures in the range from 20 to 80°C, preferably 40 to 80°C.

8. The process as claimed in one or more of claims 1 to 7, wherein the oxidation is carried out under pressures in a range from atmospheric pressure to 1.0 MPa, preferably atmospheric pressure to 0.8 MPa.

9. The process as claimed in one or more of claims 1 to 8, wherein the oxygen-containing gas mixtures have a content of up to 90% by volume, in particular 30 to 80% by volume, of inert constituents.

## Revendications

1. Procédé pour la préparation d'acides carboxyliques aliphatiques comprenant 4 à 11 atomes de carbone par oxydation des aldéhydes correspondants avec de l'oxygène ou des mélanges gazeux contenant de l'oxygène à 20 jusqu'à 100°C, **caractérisé en ce que** l'oxydation des aldéhydes purifiés est réalisée en présence de 0,1 à 5 ppm en poids d'un métal des groupes 5 à 11 du système périodique des éléments ou de la quantité correspondante d'un composé d'un tel métal ou de mélanges de ces métaux et/ou.. composés métalliques, par rapport à l'aldéhyde utilisé et en excluant l'addition de ligands multidentates qui contiennent des hétérocycles contenant de l'azote.

2. Procédé selon la revendication 1, **caractérisé en ce que** l'oxydation des aldéhydes est réalisée en présence de 0,2 à 3 ppm, en particulier de 0,5 à 2 ppm d'un métal des groupes 5 à 11 du système périodique des éléments ou de la quantité correspondante d'un composé d'un tel métal ou de mélanges de ces métaux et/ou composés métalliques, par rapport à l'aldéhyde utilisé.

3. Procédé selon la revendication 1 ou 2, **caractérisé en ce que** le métal du 5ème au 11ème groupe du système périodique des éléments est le vanadium, le chrome, le molybdène, le fer, le cobalt, le nickel, le ruthénium, le rhodium, le palladium ou le cuivre, de préférence le chrome, le fer, le nickel, le rhodium et en particulier le fer et le rhodium.

4. Procédé selon la revendication 1 ou 2, **caractérisé en ce que** les composés métalliques sont dérivés des métaux vanadium, chrome, molybdène, fer, cobalt, nickel, ruthénium, rhodium, palladium, cuivre, de préférence chrome, fer, nickel, rhodium, et en particulier fer et rhodium.

5. Procédé selon l'une ou plusieurs des revendications 1 à 4, **caractérisé en ce que** les composés métalliques sont des carboxylates, des acétylacétonates ou des composés carbonyles.

6. Procédé selon la revendication 5, **caractérisé en ce que** les métallocarboxylates sont des sels des acides carboxyliques qui sont formés comme résultat de l'oxydation des aldéhydes utilisés.

7. Procédé selon l'une ou plusieurs des revendications 1 à 6, **caractérisé en ce que** l'oxydation est réalisée à des températures dans la plage de 20 à 80°C, de préférence de 40 à 80°C.

8. Procédé selon l'une ou plusieurs des revendications 1 à 7, **caractérisé en ce que** l'oxydation est réalisée à des pressions dans une plage allant de la pression atmosphérique à 1,0 MPa, de préférence de la pression atmosphérique à 0,8 MPa.

9. Procédé selon l'une ou plusieurs des revendications 1 à 8, **caractérisé en ce que** les mélanges gazeux contenant de l'oxygène présentent une proportion de constituants inertes allant jusqu'à 90% en volume, en particulier de 30 à 80% en volume.
